# FASCICULE DE BREVET EUROPEEN

(11) **EP 1 358 179 B1**
(45) Date de publication et mention de la délivrance du brevet: **10.05.2006**
(21) Numéro de dépôt: 02706839.4
(22) Date de dépôt: 11.02.2002
(51) Int. Cl.: C07D 401/12, C07D 211/38, C07D 213/74

(54) **PROCEDE DE SYNTHESE ET INTERMEDIAIRES DE PYRIDIN-2-YL-METHYLAMINE**
VERFAHREN ZUR HERSTELLUNG UND ZWISCHENPRODUKTE VON PYRIDIN-2-YL-AMIN
SYNTHESIS METHOD AND INTERMEDIATES OF PYRIDIN-2-YL-METHYLAMINE

(30) Priorité: 09.02.2001 FR 0101784
(43) Date de publication de la demande: 05.11.2003
(73) Titulaire: PIERRE FABRE MEDICAMENT, 92100 Boulogne-Billancourt (FR)
(72) Inventeur: MAUREL, Jean-Louis, F-81100 Castres (FR); BONNAUD, Bernard, F-81090 Lagarrigue (FR); RIBET, Jean-Paul, F-81200 Mazamet (FR); VACHER, Bernard, F-81100 Castres (FR)
(74) Mandataire: Ahner, Francis
(86) Numéro de dépôt international: PCT/FR2002/000508
(87) Numéro de publication internationale: WO 2002/064585

(56) Documents cités:
- WO-A-98/22459
- MINOR ET AL.: "Synthesis of 2- and 6-Fluoronicotinamides" J. AM. CHEM. SOC., vol. 71, 1949, pages 4152-3, XP002181607
- AYI ET AL.: "Nouvelle voie de synthèse d'acides amines monofluores" TETRAHEDRON LETTERS, vol. 22, no. 16, 1981, pages 1505-8, XP002181608

## Description

La présente invention concerne un nouveau procédé de préparation de dérivés de la pyridin-2-yl-méthylamine de formule (I) 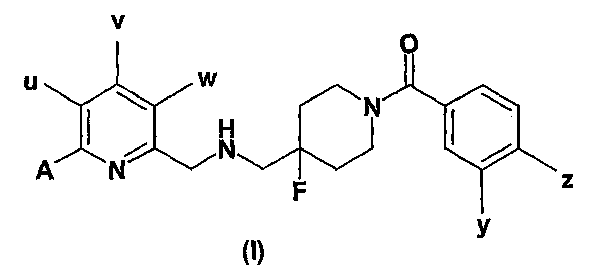 dans laquelle :
- u: représente un atome d'hydrogène ou un radical méthyle ;
- v: représente un atome d'hydrogène ou un atome de chlore ou un radical méthyle ;
- w: représente un atome d'hydrogène ou un atome de fluor ou un radical méthyle ;
- y: représente un atome de chlore ou un radical méthyle ;
- z: représente un atome d'hydrogène ou un atome de fluor ou un atome de chlore ou un radical méthyle ;
- A: représente :
- un atome d'hydrogène ou un atome de fluor ou un atome de chlore ;
- un radical alkyle en C₁-C₅ ;
- un radical fluoroalkyle ;
- un radical cyclopropyle ;
- un groupe hétérocyclique aromatique à 5 chaînons ;
- un groupe alkoxy ou alkylthio ;
- un groupe amino cyclique ;
- un groupe alkoxycarbonyle,
- un groupe amino du type : 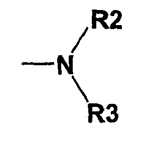
dans lequel R2 ou R3, identiques ou différents représentent l'hydrogène, ou un radical alkyl en C₁-C₅ tel que définis précédemment ou un radical cyclopropyle ou cyclobutyle ou un radical trifluorométhyle.

Le procédé est caractérisé par la réaction d'une cyanohydrine de formule (III) : 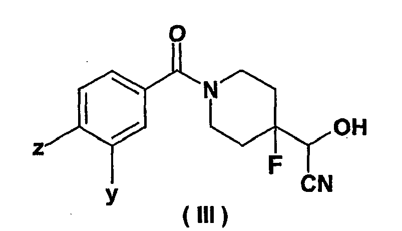 et d'une pyridin-2-yl-méthylamine de formule (IV) :

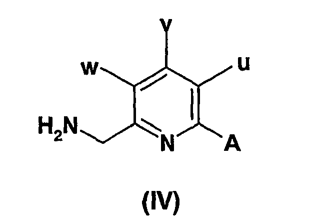

Selon un mode de mise en oeuvre particulier de l'invention, le milieu réactionnel peut dans certains cas, être avantageusement rendu basique par l'addition d'une base organique de type amine tertiaire. On utilisera en particulier le 1,4-diazabicyclo [2,2,2] octane.

Selon une autre caractéristique de mise en oeuvre du procédé de l'invention, le milieu réactionnel est rendu réducteur par l'addition d'un hydrure de bore, simple ou complexe, en particulier le cyanoborohydrure de sodium.

Selon une caractéristique additionnelle de la présente invention, un tel procédé sera avantageusement conduit dans un milieu réactionnel de type alcoolique, en particulier en milieu méthanolique.

Selon une autre caractéristique additionnelle du procédé de l'invention, l'amine primaire, réactif de départ de formule (IV), pourra être utilisée sous la forme d'un chlorhydrate et ceci plus particulièrement, dans le cas de la préparation d'une pyridin-2-yl-méthylamine.

Ces composés sont utiles comme médicaments notamment comme antidépresseurs et analgésiques.

Dans les techniques antérieures, illustrées dans WO 9822459, les composés de formule (I) sont obtenus à partir d'une 1-benzoyl-pipéridin-4-one (formule II). La fonction cétone des composés de formule (II) est transformée en époxyde (IIa), qui traité par un excès du complexe fluorure d'hydrogène-pyridine, conduit à une 1-benzoyl-4-fluoro-4-hydroxyméthyl-pipéridine (IIb). La fonction alcool primaire de ce composé (IIb) est ensuite activée sous forme d'ester d'acide para-toluènesulfonique (IIc) pour donner après réaction avec le phtalimide potassique une 1-benzoyl-4-fluoro-4-(1-phtalimidoylméthyl)-pipéridine (IId). Cet intermédiaire, après traitement au moyen d'éthanolamine conduit à une 1-benzoyl-4-fluoro-4-aminométhyl-pipéridine (IIe), qui est ensuite engagée dans une réaction d'amination réductrice avec un aldéhyde approprié pour donner les composés de formule (I).

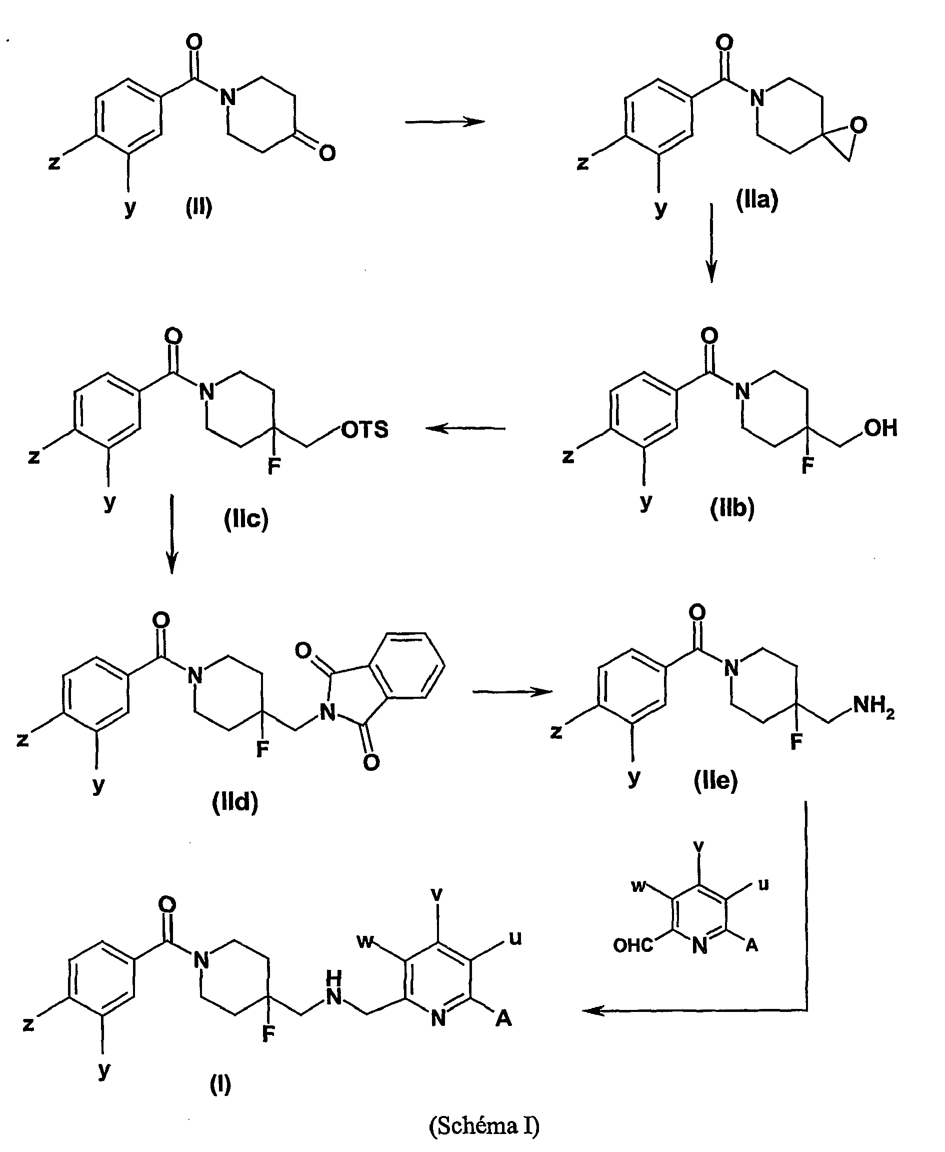

L'obtention des composés de formule (I) selon le procédé, tel qu'il est décrit dans WO 9822459, nécessite 6 étapes à partir de l'intermédiaire de formule (II) et fournit un rendement global optimisé moyen de 5%.

Le présent procédé qui utilise une nouvelle réaction d'amination réductrice entre une cyanohydrine (formule III) et une pyridin-2-yl-méthylamine (formule IV), remplace avantageusement le procédé antérieur.

Le procédé utilisant cette nouvelle réaction est décrit dans le schéma II :

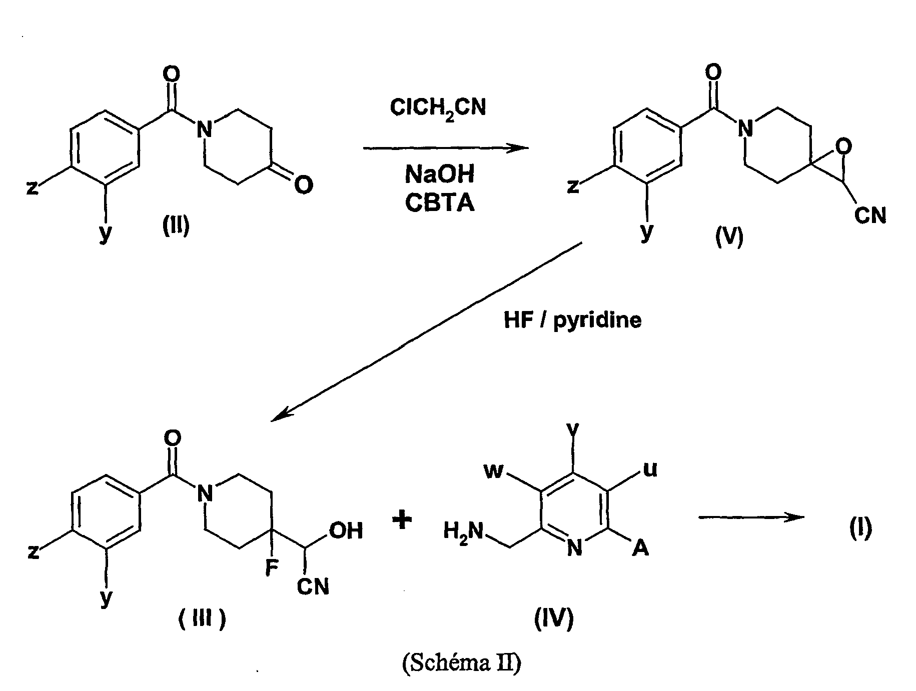

Une réaction de Darzens entre les benzoyl-pipéridin-4-ones (II) et un halogénure d'acétonitrile (A. Jonczyk, Tetrahedron Lett. (1972), 23, 2395-96) donne les cyanoépoxydes correspondants (formule V). Cette réaction est effectuée avantageusement selon une technique de transfert de phase dans des conditions opératoires développées par les inventeurs. L'ouverture de l'époxyde (V) au moyen d'acide fluorhydrique ou autres agents de fluoration, selon les techniques décrites par exemple dans :
J. Fluorine Chem. (1999), 99(2), 95-97
Yuki Gosei Kagaku Kyokaishi (1998), 56(4), 312-319
J. Fluorine Chem.(1995), 70(1), 1-3
J. Fluorine Chem. (1995), 70(1), 141-4
Tetrahedron Lett. (1990), 31(49), 7209-12
J. Chem. Soc., Chem. Commun. (1989), (23), 1848-50
conduit aux cyanohydrines de formule (III). Ces dernières sont alors mises en réaction avec les pyridin-2-yl-méthylamines (formule IV) ou leur sel, en milieu réducteur et éventuellement basique pour donner directement les composés de formule (I). Le rendement global moyen de ce procédé, à partir de l'intermédiaire de formule (II), est de 23%, ce qui représente un gain de productivité de 450% par rapport au procédé initial (schéma I).

Les rendements de cette nouvelle réaction, appliquée à la cyanohydrine (III), (schéma II), et l'amine primaire (IV), ainsi que la pureté des produits obtenus, permettent donc une synthèse industrielle plus économique et efficace.

La réaction d'amination réductrice est avantageusement conduite à température ambiante.

Lorsque les pyridine-2-yl-méthylamines se présentent sous la forme de sel, alors, le milieu réactionnel est avantageusement rendu basique au moyen d'une base organique, type amine tertiaire, tel que, par exemple, le 1,4-diazabicyclo[2,2,2]octane (DABCO).

Le milieu réducteur est avantageusement obtenu au moyen d'un hydrure de bore, simple ou complexe, ou d'un mélange d'hydrures de bore et, en particulier, de cyanoborohydrure de sodium (NaBH₃CN).

Dans certains cas, notamment lorsque la (6-aminométhyl-5-méthyl-pyridin-2-yl)-méthylamine (formule IVb) est utilisée, il apparaît un produit secondaire de formule (VII), (schéma III).

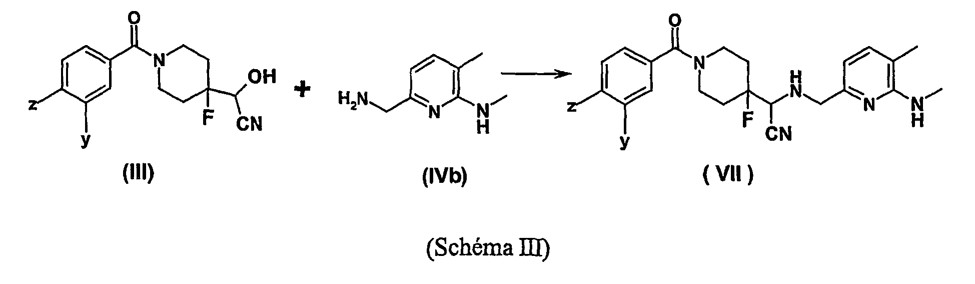

Cette réaction secondaire, peut être supprimée par addition de sulfate de fer (FeSO₄, 7H₂O au milieu réactionnel. D'autres sels de métaux capables de complexer les ions cyanures peuvent être également utilisés dans la réaction d'amination réductrice en question.

La préparation de la (5-méthyl-pyridin-2yl)-méthylamine (formule IVa) utilisée dans ce procédé est décrite dans EP 718 300, US 4 482 437 ou page 692 de l'article de Davies et al (J. Chem. Soc ; 1970, pages 688-693).

Là préparation de la (6-aminométhyl-5-méthyl-pyridin-2-yl)-méthylamine (formule IVb), est décrite ci-après et a été conduite selon la séquence de réactions représentées dans le schéma IV.

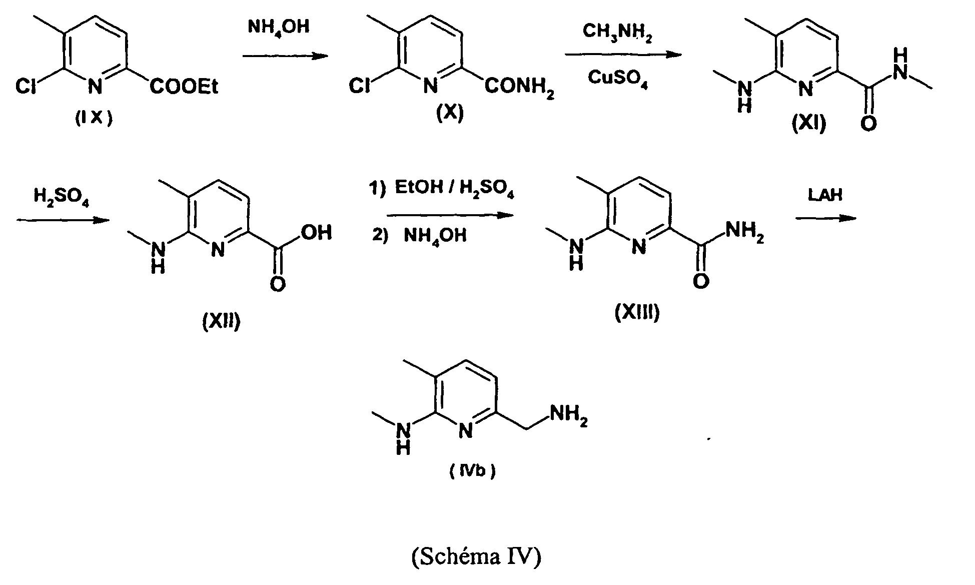

La préparation de la 2-éthoxycarbonyl-5-méthyl-6-chloro-pyridine (composé IX) est décrite dans WO 9822459. Cet intermédiaire traité à l'ammoniaque donne le dérivé 2-carboxamido-5-méthyl-6-chloro-pyridine (formule X) qui réagit à chaud et sous pression avec de la méthylamine en présence de sulfate de cuivre pour donner la 2-N-méthylamido-5-méthyl-6-méthylamino-pyridine (formule XI). L'hydrolyse acide de l'amide, suivie d'une estérification et d'un traitement à l'ammoniaque conduisent à la 2-carboxamido-5-méthyl-6-méthylamino-pyridine (formule XIII) qui est réduite en (6-arninométhyl-5-méthyl-pyridine-2-yl)-méthylamine (formule IVb) au moyen d'hydrure d'aluminium-lithium.

Les exemples suivants illustrent l'invention sans toutefois en limiter la portée.

Exemple 1 : (6-aminométhyl-5-méthyl-pyridin-2yl)-méthylamine (IVb) :

### Etape 1 : 2-carboxamido-5-méthyl-6-chloro-pyridine (IX).

Dissoudre 26 g de 2-éthoxycarbonyl-5-méthyl-6-chloro-pyridine (huile brute contenant environ 60 % d'ester) dans 130 ml de méthanol. Ajouter 200 ml d'ammoniaque 32 % et agiter une nuit à température ambiante. Le produit est récupéré par filtration et donne après lavage à l'eau et séchage sous vide 13 g de cristaux blancs Tf=146°C.

### Etape 2 : 2-méthylcarboxamido-5-méthyl-6-méthylamino-pyridine (XI).

Dans un réacteur étanche en inox, on introduit 73 g de 2-carboxamide-5-méthyl-6-choro-pyridine, 200 ml de méthylamine à 40 % dans l'éthanol , 110 ml de méthylamine à 40 % dans l'eau et 34 g de sulfate de cuivre anhydre. Le réacteur est ensuite fermé et chauffé sous agitation 24 heures à 110°C. Après refroidissement, le milieu réactionnel est dilué avec 300 ml d'eau et de l'ammoniaque à 32 %. Le produit est extrait 2 fois au dichlorométhane puis lavé à l'eau salée. Après séchage et évaporation, on récupère 52,4 g de cristaux blancs Tf= 158°C.

### Etape 3 : acide 5 méthyl-6-méthylamino-pyridine-2-carboxylique (XII).

Dans une solution de 410 ml d'acide sulfurique à 95 % dans 100 ml d'eau, on ajoute 52 g de 2-méthylamino-5-méthyl-6-méthylamino-pyridine et l'on porte le mélange 48 heures à 100°C. Après refroidissement, le milieu réactionnel est versé sur de la glace et neutralisé avec de l'ammoniaque. L'eau est évaporée sous vide et le résidu est repris dans du méthanol. Après filtration des minéraux, l'évaporation du méthanol fournit 57 g d'une masse brune solide qui sera utilisée dans l'étape suivante sans autre purification.

### Etape 4 : 2-carboxamido-5-méthyl-6-méthylamino-pyridine (XIII).

50 g du solide obtenu précédemment sont repris dans 1,5 1 d'éthanol additionné de 38 ml d'acide sulfurique concentré. Le mélange est chauffé au reflux du solvant pendant 24 heures. Après refroidissement, 1 litre d'ammoniaque 32 % est ajouté et le mélange porté 4 heures à 50°C. L'éthanol est évaporé et le résidu additionné de 100 ml d'eau salée est extrait 10 fois au dichlorométhane puis les phases organiques sont lavées à la soude N, séchées sur MgSO₄ et évaporées à sec. Les cristaux sont lavés à l'éther puis séchés pour donner 20,5 g de cristaux blanc. Tf =182°C.

### Etape 5 : (6-aminométhyl-5-méthyl-pyridin-2-yl)-méthylamine (IVb)

11,9 g de 2-carboxamido-5-méthyl-6-méthylamino-pyridine sont mis en solution dans 60 ml de tétrahydrofurane. On introduit ensuite lentement 143 ml d'une solution d'hydrure d'aluminium lithium 1 M dans le tétrahydrofurane. La solution est chauffée au reflux du solvant pendant 4 heures, puis refroidie au bain de glace. On introduit alors goutte à goutte 5 ml d'eau puis 3,75 ml de soude 20 % et enfin 35 ml d'eau. Le solide blanc est séparé par filtration et la phase organique est évaporée à sec. Le résidu est chromatographié sur silice 60 avec un mélange de dichlorométhane 90 - méthanol 9 - ammoniaque 1, pour donner 8,4g d'huile jaune. RMN ¹H (DMSOd₆) : δ 7.12 (d, *J*= 7,16 H_{z}, 1 H) - 6,42 (d, *J=* 7,16 Hz, 1 H) - 5,80 (m, 1 H) - 3,62 (s, 2 H) - 2,82 (d, *J*= 4,8 Hz, 3 H)- 1,99 (s, 3 H) - 1.5 - 2,1 (large, 2 H).

### Exemple 2 : [1-(3-chloro-4-fluoro-benzoyl)-4-fluoro-pipéridin-4-yl]-hydroxy-acétonitrile (III).

### Etape 1 : 6-(3-chloro-4-fluoro-benzoyl)-1-oxa-6-aza-spiro[2.5]octane-2-carbonitrile (V).

Une suspension de 1-(3-chloro-4-fluoro-benzoyl)-pipéridin-4-one (4160g, 16,27 mol) dans 28,4 litres de dichlorométhane et 11,7 litres de soude à 30,5%, additionnée de 186g de chlorure de tétrabutylammonium, est refroidie à 15°C. On additionne ensuite lentement et sous forte agitation le chloroacétonitrile (1540 ml, 24,4 mol) et le mélange est agité 3 heures à 20°C. Une addition complémentaire de chloroacétonitrile (500 ml) est effectuée pour terminer la réaction. Le milieu réactionnel est dilué avec du dichlorométhane (8,5 litres) et de l'eau (20 litres) puis décanté et relavé à l'eau. La solution brune obtenue est décolorée par 2 Kg de silice et 500 g de noir animal puis évaporée à sec. Le résidu obtenu est cristallisé dans l'isopropanol pour donner après filtration 3426 g de cristaux bruns.
Tf= 100-101°C.

### Etape 2: [1-(3-chloro-4-fluoro-benzoyl)-4-fluoro-pipéridin-4-yl]-hydroxy-acétonitrile (III).

Dans un réacteur en hastelloy, équipé d'un laveur de gaz approprié (vapeurs HF), on introduit le 6-(3-chloro-4-fluoro-benzoyl)-1-oxa-6-aza-spiro[2.5]octane-2-carbonitrile (2620 g, 8,89 mol) en solution dans du dichlorométhane (6,6 litres). La solution est amenée à 15 °C et l'on introduit 2.91 Kg du complexe HF/pyridine à 70% et l'on agite 6 heures à 40°C. Le milieu réactionnel est ensuite lavé 2 fois avec 10 litres d'eau puis avec une solution de K₂CO₃ et enfin à l'eau. La phase organique est évaporée sous vide et le résidu cristallisé dans 10 litres d'isopropanol. La filtration et le séchage sous vide du précipité fournissent 1540 g de cristaux blancs. Tf=139-140°C.

### Exemple 3 : (3-Chloro-4-fluoro-phényl)-(4-fluoro-4-{[(5-méthyl-pyridine-2-ylméthyl)-amino]-méthyl}-pipéridin-1yl)-méthanone (Ia).

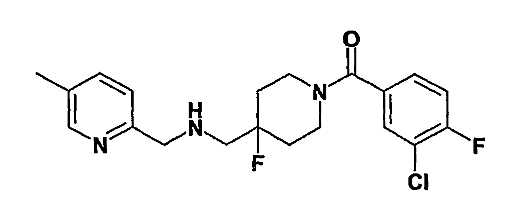

Dans un ballon, introduire le [1-3-Chloro-4-fluoro-benzoyl)-4-pipéridin-4-yl]-hydroxy-acetonitrile (5,3 g, 0.0169 mol), la (5-méthyl-pyridin-2-yl)-méthylamine (dichlorhydrate) (3,6 g, 0.0185 mol), le 1,4-diazabicylo[2,2,2]octane (6,2 g, 0.055 mol), le cyanoborohydrure de sodium (1,25 g, 0.02 mol) et 150 ml de méthanol. Le tout est agité 4 heures à température ambiante, puis évaporé à sec. Le résidu est repris dans l'eau bicarbonatée et extrait à l'acétate d'éthyle, les phases organiques sont lavées à l'eau puis séchées sur MgSO₄ et évaporées. Le résidu est chromatographié sur silice 60 avec un mélange de dichlorométhane 95 - méthanol 4,5 - ammoniaque 0,5 pour donner 5,22 g d'huile (78 %). Cette huile est ensuite traitée dans l'acétate d'éthyle avec un équivalent d'acide fumarique pour donner le sel cristallin blanc Tf=157°C.
MS : DCI > 0 MH⁺ m/z = 394.
Analyse élémentaire :
calculé : C 56,53 %, H 5,14 %, N 8,24 % ,
Trouvé C 56,67 %, H 5,21 %, N 8,41 %.
RMN ¹H (DMSOd₆) : δ 10,4 - 9,4 (large, 3 H) - 8,4 (dd, ⁴*J =* 1,40 Hz - ⁵*J*= 0,83 Hz, 1 H) - 7,7 (dd, ⁴*J*= 7,18 Hz - ⁴*J=* 1.93 Hz, 1 H) - 7,6 (dd, ³*J=* 8 Hz, ⁴*J=* 1,5Hz, 1 H) - 7,50 (dd ³*J*= 8,6 Hz, ³*J*= 8,6 Hz, 1 H)- 7,45 (ddd, ³*J*= 8,6 Hz, ⁴*J*= 5 Hz, ⁴*J*= 1,93 Hz, 1 H) - 7,35 (d ³*J* = 8 Hz, 1 H) - 6,61 (s, 2 H) - 4,40 - 4,20 (large, 1 H) - 3,92 (s, 2 H) - 3,50 - 3,30 (large, 1 H) - 3,30 - 3,20 (large, 1 H) - 3,15 - 2,95 (large, 1 H) - 2,83 (d, ³*J*= 20,7 Hz, 2 H) - 2,29 (s, 3 H) - 2,07 - 1,90 (large, 1 H) - 1,90 - 1,80 (large, 1 H) - 1,83 - 1,77 (m, 1 H) - 1,75 - 1,60 (m, 1 H).

### Exemple 4 : (3-Choro-4-fluoro-phényl)-4-fluoro-4-{[(5-méthyl-6-méthylamino-pyridin-2-ylméthyl)-amino]-méthyl}-pipéridin-1-yl)-méthamine (Ib).

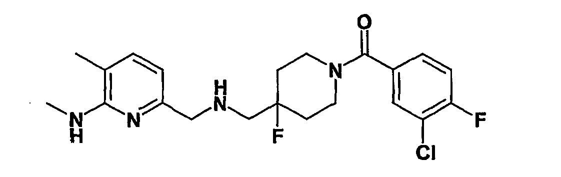

Dans un ballon, introduire le [1-(3-Chloro-4-fluoro-benzoyl)-4-fluoro-pipéridin-4-yl]-hydroxy-acetonitrile (11,6 g, 0,037 mol), la (6-méthylamino-5-méthyl-pyridine-2-yl)-méthylamine (6,8 g, 0,045 mol), le 1,4 diazabyciclo[2,2,2]octane (9,1 g, 0,081 mol), le cyanoborohydrure de sodium (3,8 g, 0,06 mol), le FeSO₄, 7H₂O (11,3 g, 0,0407 mol) et 300 ml de méthanol. Le tout est agité 4 heures à température ambiante puis évaporé à sec. Le résidu est repris dans de l'eau et extrait 3 fois à l'acétate d'éthyle, les phase organiques sont lavées à l'eau puis à l'eau salée et séchées sur MgSO₄. L'évaporation du solvant fournit une huile qui est ensuite chromatographiée sur silice 60 avec : dichlorométhane 95 - méthanol 4,5 - ammoniaque 0,5 pour donner 14,8 g de base (94 %). La salification dans l'acétate d'éthyle avec un équivalent d'acide glycolique fournit un sel cristallin blanc.
Tf=122°C
MS : ESI>O MH⁺ m/z = 423.
Analyse élémentaire :
Calculé : C 55,37 % - H 5,86 % - N 11,23 %
Trouvé : C 55,17 %, H 5,99 %, N 11,08 %.
RMN ¹H (D₂O): δ 7,61 (dd, ⁴*J=* 2 Hz - ⁴*J=* 7 Hz, 1 H) - 7,47 (d ³*J* = 7,1 Hz, 1 H) - 7,45 (m, 1 H) - 7,39 (dd, ³*J* = 8,6 Hz - ³*J*= 8,6 Hz, 1 H) - 6,73 (d, ³*J*= 7,2 Hz, 1 H) - 4,94 (s, HOD) - 4,55 - 4,45 (d, ²*J=* 11 Hz, 1 H) - 4,28 (s, 2H) - 4,01 (s, 2H) - 3,70 - 3,80 (d, ²*J*= 11 Hz, 1 H) - 3,45 - 3,60 (dd, ²*J=* 11Hz - ³*J=* 11 Hz, 1 H) - 3,35 - 3,25 (m, 1 H) - 3,35 (d, ³*J=* 20,5 Hz, 2 H) - 3,03 (s, 3 H) - 2,30 - 2,15 (dd, ²*J=* 10 Hz - ³*J=* 10 Hz, 1 H) - 2,17 (s, 3 H) - 2,10 - 1,90(m,1 H) - 1,95 - 1,80 (m, 1 H)- 1,80 - 1,70 (m, 1 H).

## Revendications

1. Procédé de préparation des dérivés des pyridin-2-yl-méthylamines de formule (I) 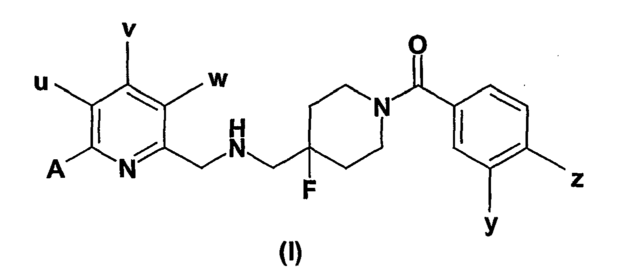 dans laquelle :
u représente un atome d'hydrogène ou un radical méthyle ;
v représente un atome d'hydrogène ou un atome de chlore ou un radical méthyle ;
w représente un atome d'hydrogène ou un atome de fluor ou un radical méthyle ;
y représente un atome de chlore ou un radical méthyle ;
z représente un atome d'hydrogène ou un atome de fluor ou un atome de chlore ou un radical méthyle ;
A représente :
- un atome d'hydrogène ou un atome de fluor ou un atome de chlore ;
- un radical alkyle en C₁-C₅ ;
- un radical fluoroalkyle ;
- un radical cyclopropyle ;
- un groupe hétérocyclique aromatique à 5 chaînons ;
- un groupe alkoxy ou alkylthio ;
- un groupe amino cyclique ;
- un groupe alkoxycarbonyle.
- un groupe amino du type : 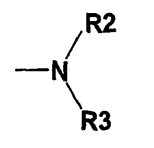
dans lequel R2 ou R3, identiques ou différents représentent l'hydrogène, ou un radical alkyl en C₁-C₅ tel que définis précédemment ou un radical cyclopropyle ou cyclobutyle ou un radical trifluorométhyle,
**caractérisé par** la mise en oeuvre de cyanohydrines de formule (III) 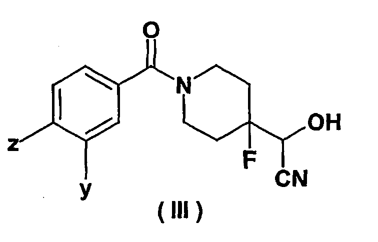 et de pyridin-2-yl-méthylamines de formule (IV) 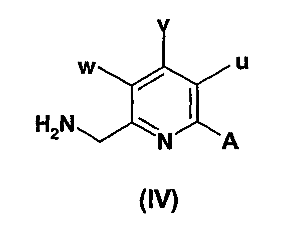 dans lesquelles les radicaux u, v, w, y, z et A ont les significations données ci-dessus à propos de la formule (I).

2. Procédé selon la revendication 1 **caractérisé en ce que** le milieu réactionnel est rendu réducteur par l'addition d'un hydrure de bore, simple ou complexe.

3. Procédé selon la revendication 2 **caractérisé en ce que** l'hydrure utilisé est le cyanoborohydrure de sodium.

4. Procédé selon l'une des revendications 1 à 3 **caractérisé par** l'addition d'un sel métallique de fer, de cuivre ou de zinc en tant que piégeur d'ions cyanures.

5. Procédé selon la revendication 4 **caractérisé en ce que** le sel utilisé est le sulfate de fer (II), FeSO₄, 7H₂O.

6. Procédé selon l'une des revendications 1 à 5 **caractérisé en ce que** le composé de formule (IV) utilisé est la (5-méthyl-pyridin-2-yl)-méthylamine de formule :

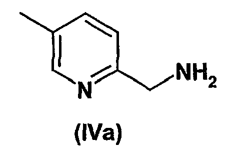

7. Procédé selon l'une des revendications 1 à 6 **caractérisé en ce que** le composé de formule (IV) utilisé est la (6-méthylamino-5-méthyl-pyridin-2-yl)-méthylamine de formule :

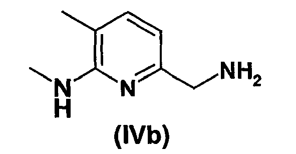

8. Procédé de préparation de la (6-méthylamino-5-méthyl-pyridin-2-yl)-méthylamine de formule (IVb) **caractérisé par** la mise en oeuvre des réactions suivantes :
- transformation de la 2-éthoxycarbonyl-5-méthyl-6-pyridine de formule (IX) en 2-carboxamido-5-méthyl-6-chloro-pyridine de formule (X) au moyen d'ammoniaque 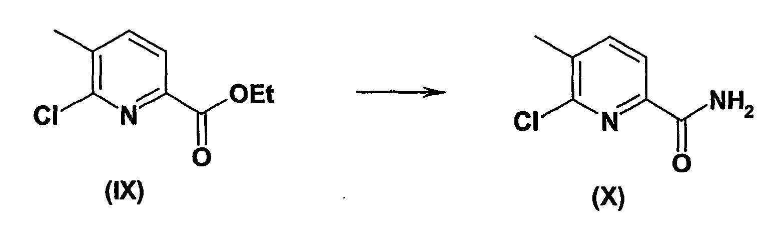
- traitement de ce composé par la méthylamine en solution et en présence de CuSO₄ à température et pression élevées pour obtenir la 2-méthylamido-5-méthyl-6-méthylamino-pyridine de formule (XI) 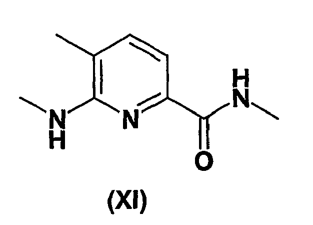
- l'hydrolyse acide de ce composé conduisant à l'acide-5-méthyl-6-méthylamino-pyridine-2-carboxylique de formule (XII) 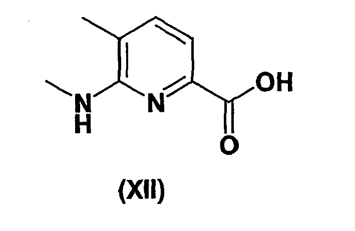
- la transformation de cet acide en 2-carboxamido-5-méthyl-6-méthylammo-pyridine de formule (XIII) 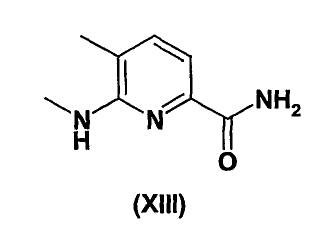
- la réduction au moyen d'hydrure d'aluminium lithium de cet amide pour conduire à la (6-méthylamino-5-pyridin-2-yl)-méthylamine de formule (IVb).

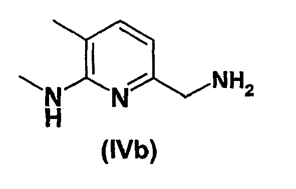

9. Procédé de préparation des (1-benzoyl-4-fluoro-pipéridin-4-yl)-hydroxy-acétonitriles de formule (III) précités **caractérisé par** la mise en oeuvre des réactions suivantes :
- réaction d'un halogénure d'acétonitrile, tel que par exemple, le chloroacétonitrile sur une 1-benzoyl-pipéridin-4-one (formule II), pour conduire au cyanoépoxyde correspondant (formule V) 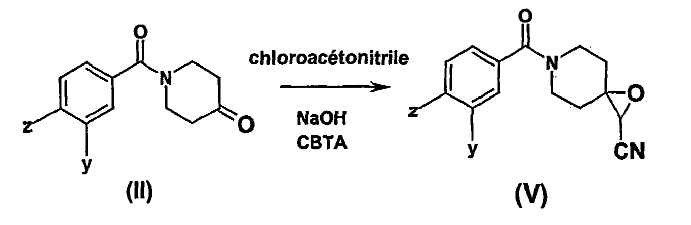
- ouverture du cyanoépoxyde précité au moyen d'un agent de fluoration, tel que par exemple, le complexe fluorure d'hydrogène-pyridine : 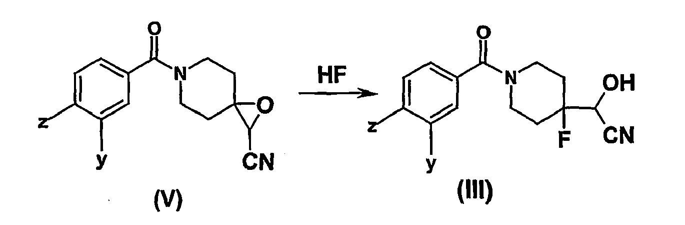
où y et z sont tels que définis précédemment.

10. Nouveaux intermédiaires de synthèse de formule générale (III) utilisés pour la préparation des composés de formule générale (I) 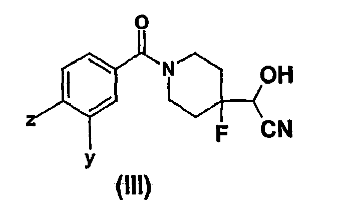 où y et z sont tels que définis précédemment.

## Patentansprüche

1. Verfahren zur Herstellung von Derivaten von Pyridin-2-ylmethylaminen der Formel (I) 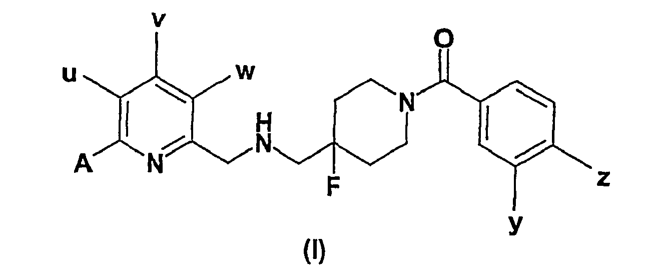 in der:
u ein Wasserstoffatom oder einen Methylrest darstellt;
v ein Wasserstoffatom oder ein Chloratom oder einen Methylrest darstellt;
w ein Wasserstoffatom oder ein Fluoratom oder einen Methylrest darstellt;
y ein Chloratom oder einen Methylrest darstellt;
z ein Wasserstoffatom oder ein Fluoratom oder ein Chloratom oder einen Methylrest darstellt;
A darstellt:
- ein Wasserstoffatom oder ein Fluoratom oder ein Chloratom;
- einen (C₁-C₅)-Alkylrest;
- einen Fluoralkylrest;
- einen Cyclopropylrest;
- eine heterocyclische aromatische Gruppe mit 5 Ringgliedern;
- eine Alkoxy- oder Alkylthiogruppe;
- eine cyclische Aminogruppe;
- eine Alkoxycarbonylgruppe;
- eine Aminogruppe des Typs: 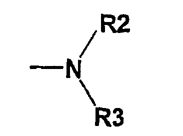
worin R2 oder R3, identisch oder verschieden, Wasserstoff oder einen (C₁-C₅)-Alkylrest, wie vorstehend definiert, oder einen Cyclopropyl- oder Cyclobutylrest oder einen Trifluormethylrest darstellen,
**gekennzeichnet durch** den Einsatz von Cyanhydrinen der Formel (III) 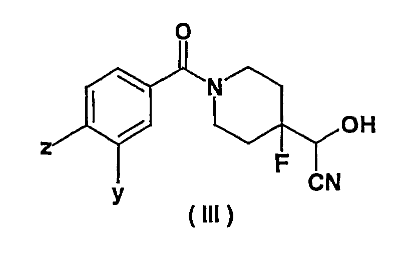 und von Pyridin-2-ylmethylaminen der Formel (IV) 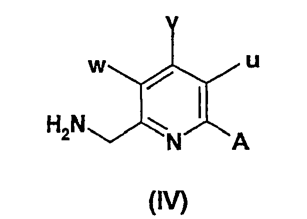 worin die Reste u, v, w, y, z und A die vorstehend mit Bezug auf die Formel (I) angegebenen Bedeutungen aufweisen.

2. Verfahren nach Anspruch 1, **dadurch gekennzeichnet, dass** das Reaktionsmedium durch Zugabe eines einfachen oder komplexen Borhydrids reduzierend gemacht wird.

3. Verfahren nach Anspruch 2, **dadurch gekennzeichnet, dass** das verwendete Hydrid Natriumcyanoborhydrid ist.

4. Verfahren nach einem der Ansprüche 1 bis 3, **gekennzeichnet durch** die Zugabe eines Metallsalzes von Eisen, Kupfer oder Zink als Abfänger von Cyanidionen.

5. Verfahren nach Anspruch 4, **dadurch gekennzeichnet, dass** das verwendete Salz Eisen(II)-sulfat, FeSO₄, 7H₂O, ist.

6. Verfahren nach einem der Ansprüche 1 bis 5, **dadurch gekennzeichnet, dass** die verwendete Verbindung der Formel (IV) (5-Methylpyridin-2-yl)methylamin der Formel: 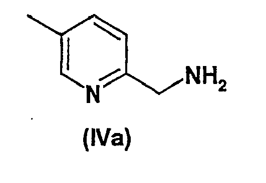 ist.

7. Verfahren nach einem der Ansprüche 1 bis 6, **dadurch gekennzeichnet, dass** die verwendete Verbindung der Formel (IV) (6-Methylamino-5-methylpyridin-2-yl)methylamin der Formel: 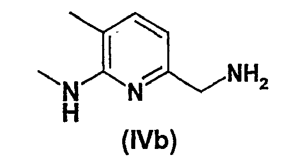 ist.

8. Verfahren zur Herstellung von (6-Methylamino-5-methylpyridin-2-yl)methylamin der Formel (IVb), **gekennzeichnet durch** die Durchführung der folgenden Reaktionen:
- Überführung von 2-Ethoxycarbonyl-5-methyl-6-pyridin der Formel (IX) in 2-Carboxamido-5-methyl-6-chlorpyridin der Formel (X) mittels Ammoniak 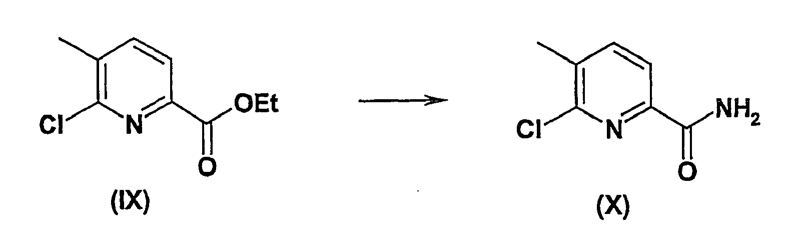
- Behandlung dieser Verbindung mit Methylamin in Lösung und in Anwesenheit von CuSO₄ bei erhöhter Temperatur und erhöhtem Druck, um 2-Methylamido-5-methyl-6-methylaminopyridin der Formel (XI) zu erhalten 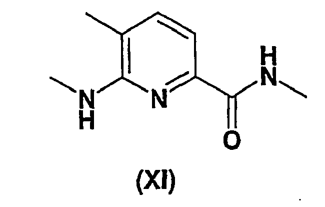
- saure Hydrolyse dieser Verbindung, die zu 5-Methyl-6-methylaminopyridin-2-carbonsäure der Formel (XII) führt 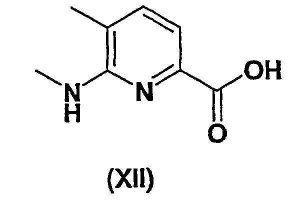
- Überführung dieser Säure in 2-Carboxamido-5-methyl-6-methylaminopyridin der Formel (XIII) 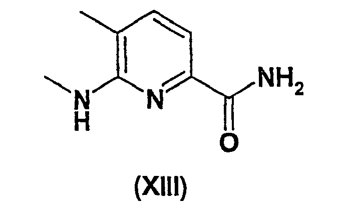
- Reduktion dieses Amids mittels Lithiumaluminiumhydrid, um zu (6-Methylamino-5-pyridin-2-yl)methylamin der Formel (IVb) zu gelangen.

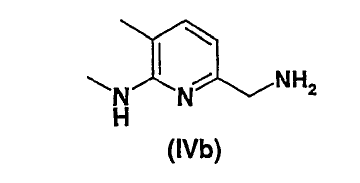

9. Verfahren zur Herstellung von (1-Benzoyl-4-fluorpiperidin-4-yl)hydroxyacetonitrilen der vorstehenden Formel (III), **gekennzeichnet durch** die Durchführung der folgenden Reaktionen:
- Umsetzung eines Acetonitrilhalogenids, wie zum Beispiel Chloracetonitril, mit einem 1-Benzoylpiperidin-4-on (Formel II), was zum entsprechenden Cyanoepoxid (Formel V) führt 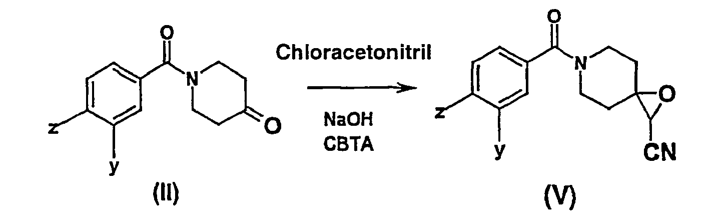
- Öffnen des genannten Cyanoepoxids mittels eines Fluorierungsmittels, wie zum Beispiel dem Fluorwasserstoff-Pyridin-Komplex: 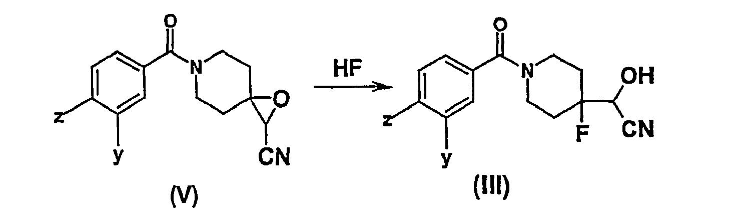
- worin y und z wie vorstehend definiert sind.

10. Neue Synthesezwischenprodukte der allgemeinen Formel (III), die für die Herstellung von Verbindungen der allgemeinen Formel (I) verwendet werden 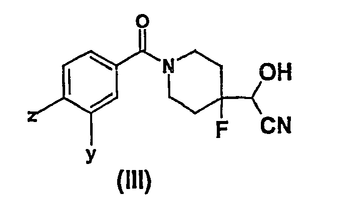 worin y und z wie vorstehend definiert sind.

## Claims

1. Method for preparing pyridin-2-yl-methylamine derivatives of formula (I) 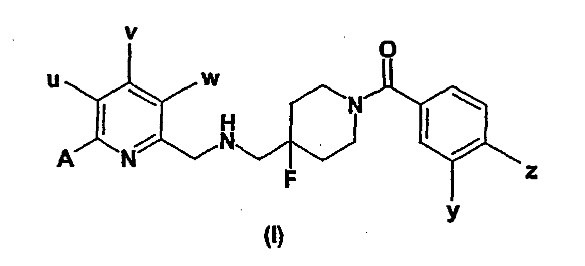 in which:
u represents a hydrogen atom or a methyl radical;
v represents a hydrogen atom or a chlorine atom or a methyl radical;
w represents a hydrogen atom or a fluorine atom or a methyl radical;
y represents a chlorine atom or a methyl radical;
z represents a hydrogen atom or a fluorine atom or a chlorine atom or a methyl radical;
A represents:
- a hydrogen atom or a fluorine atom or a chlorine atom;
- a C₁-C₅ alkyl radical;
- a fluoroalkyl radical;
- a cyclopropyl radical;
- a 5-membered aromatic heterocyclic group;
- an alkoxy or alkylthio group;
- a cyclic amino group;
- an alkoxycarbonyl group;
- an amino group of the type: 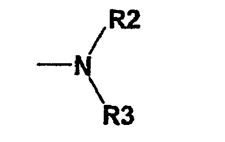
in which R2 or R3, which are identical or different, represent hydrogen, or a C₁-C₅ alkyl radical as defined above or a cyclopropyl or cyclobutyl radical or a trifluoromethyl radical,
**characterized by** the use of cyanohydrins of formula (III) 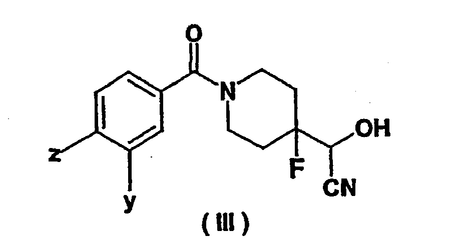 and of pyridin-2-ylmethylamines of formula (IV) 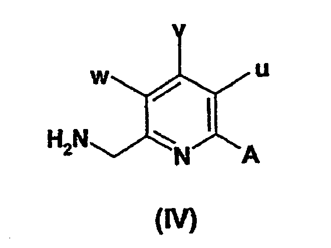 in which the radicals u, v, w, y, z and A have the meanings given above with respect to formula (I).

2. Method according to Claim 1, **characterized in that** the reaction medium is made reducing by the addition of a simple or complex boron hydride.

3. Method according to Claim 2, **characterized in that** the hydride used is sodium cyanoborohydride.

4. Method according to one of Claims 1 to 3, **characterized by** the addition of a metal salt of iron, copper or zinc as a scavenger of cyanide ions.

5. Method according to Claim 4, **characterized in that** the salt used is the iron(II) sulphate, FeSO₄.7PH₂O.

6. Method according to one of Claims 1 to 5, **characterized in that** the compound of formula (IV) used is (5-methylpyridin-2-yl)methylamine of formula:

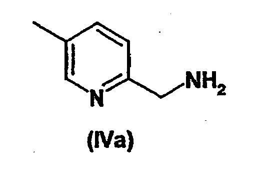

7. Method according to one of Claims 1 to 6, **characterized in that** the compound of formula (IV) used is (6-methylamino-5-methylpyridin-2-yl)-methylamine of formula:

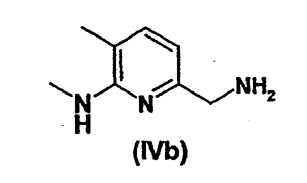

8. Method for preparing (6-methylamino-5-methyl-pyridin-2-yl)methylamine of formula (IVb), **characterized by** the use of the following reactions:
- conversion of the 2-ethoxycarbonyl-5-methyl-6-pyridine of formula (IX) to 2-carboxamido-5-methyl-6-chloropyridine of formula (X) by means of aqueous ammonia 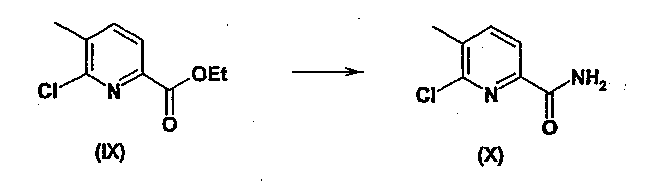
- treatment of this compound with methylamine in solution and in the presence of CuSO₄ at high temperature and pressure in order to obtain the 2-methylamido-5-methyl-6-methylaminopyridine of formula (XI) 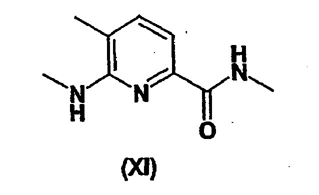
- acid hydrolysis of this compound leading to the 5-methyl-6-methylaminopyridine-2-carboxylic acid of formula (XII) 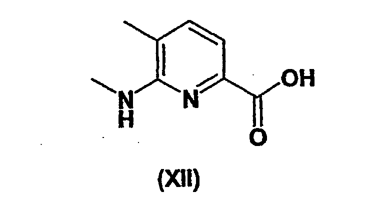
- conversion of this acid to 2-carboxamido-5-methyl-6-methylaminopyridine of formula (XIII) 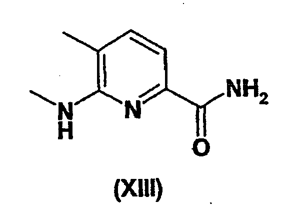
- reduction by means of lithium aluminium hydride of this amide to give the (6-methylamino-5-pyridin-2-yl)methylamine of formula (IVb).

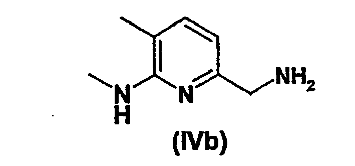

9. Method for preparing the (1-benzoyl-4-fluoro-piperidin-4-yl)hydroxyacetonitriles of formula (III) mentioned above, **characterized by** the use of the following reactions:
- reaction of an acetonitrile halide, such as for example chloroacetonitrile, with a 1-benzoyl-piperidin-4-one (formula II) to give the corresponding cyanoepoxide (formula V) 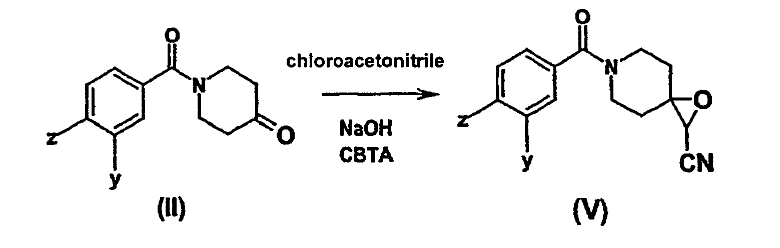
- opening of the abovementioned cyanoepoxide by means of a fluorinating agent such as for example the hydrogen fluoride-pyridine complex: 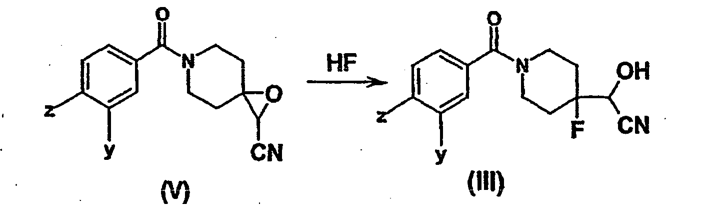
where y and z are as defined above.

10. Novel synthesis intermediate of general formula (III), used for the preparation of the compounds of general formula (I) 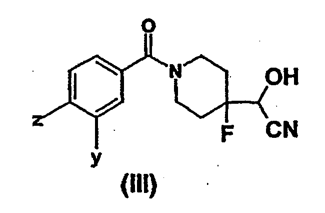 where y and z are as defined above.
